# EUROPEAN PATENT SPECIFICATION

(11) **EP 2 896 426 B1**
(45) Date of publication and mention of the grant of the patent: **29.05.2019**
(21) Application number: 15151790.1
(22) Date of filing: 20.01.2015
(51) Int. Cl.: H01B 5/10, A61M 25/09, D07B 1/06

(54) **Stranded wire and guidewire employing the same**
Litzendraht und Führungsdraht damit
Fil toronné et fil-guide l'utilisant

(30) Priority: 20.01.2014 JP 2014007794
(43) Date of publication of application: 22.07.2015
(73) Proprietor: Asahi Intecc Co., Ltd., Seto-shi, Aichi 489-0071 (JP)
(72) Inventor: Murata, Satoru c/o ASAHI INTECC CO., LTD., Seto-shi, Aichi 489-0071 (JP); Koike, Tadahiro c/o ASAHI INTECC CO., LTD., Seto-shi, Aichi 489-0071 (JP)
(74) Representative: Winter, Brandl, Fürniss, Hübner, Röss, Kaiser, Polte - Partnerschaft mbB

(56) References cited:
- EP-A1- 2 005 988
- EP-A2- 1 243 283
- US-A1- 2001 009 982
- US-A1- 2012 004 493

## Description

### [Technical Field]

The present invention relates to a medical stranded wire and a guidewire employing the stranded wire.

### [Background Art]

Conventionally, various stranded wires used for a medical apparatus have been proposed for treatment or examination (such as a stent inserted into body tissue or a tubular organ, such as a blood vessel, the digestive tract, or the ureter) . For example, Patent Literature 1 discloses a stranded wire (coiled body) composed of a plurality of element wires stranded together. The element wires forming this stranded wire are formed of Ni/Ti or anNi/Ti-based alloy in order to ensure fatigue endurance for a long period of use.

### [Citation List]

### [Patent Literature]

[Patent Literature 1]
Japanese Laid-Open Patent Publication No. 2002-10587 9.

US 2012/004493 A1 discloses a stranded wire comprising a single core element wire and at least one pair of first side element wires having tensile strength greater than that of a second side element wire. The first side element wires are arranged rotation symmetrically (rotation angle of 120°) about the center of the core.

US 2001/009982 A1 discloses a stranded wire comprising a single core element wire and at least one pair of first side element wires having tensile strength greater than that of the second side element wire. The pair of the first side element wires are arranged rotation-symmetrically.

### [Summary of Invention]

### [Technical Problem]

In the case of, for example, a hard lesion, a lesion narrowed to a great degree, or an occluded lesion, sufficient torque transmission characteristics are required in a medical apparatus to enable insertion into the lesion. Additionally, when rotating a proximal part of an apparatus having a stranded wire during insertion of the apparatus into the above-described lesions, great torsional stress is applied to the stranded wire, whereby the stranded wire is deformed or broken and safety is compromised.

Even for the stranded wire described in the above Patent Literature 1, it is difficult to ensure sufficient safety when great torsional stress is applied to the medical apparatus during use. In this respect, there is room for improvement.

The present invention has been directed towards providing a stranded wire whose deformation or breakage is suppressed so that sufficient safety is ensured, and a guidewire employing the stranded wire.

### [Solution to Problem]

According to the present invention there is provided a stranded wire comprising a core comprising a single core element wire and a plurality of side element wires wound so as to cover a circumference of the core, the core and the plurality of the side element wires being stranded together, wherein the side element wires include at least one pair of first side element wires, and at least one second side element wire other than these first side element wires, wherein the pair of first side element wires is arranged point-symmetrically about the center of the core in a cross-sectional view of the stranded wire, and the core element wire and the first side element wires have tensile strength greater than that of the second side element wire.

In order to solve the above problem, a stranded wire and a guidewire employing the stranded wire may have the following features.

In a first aspect, there is provided a stranded wire composed of a core comprising at least one core element wire and a plurality of side element wires wound so as to cover a circumference of the core, the core and the plurality of the side element wires being stranded together, wherein the side element wires include at least one pair of first side element wires arranged point-symmetrically about the center of the core in a cross- sectional view of the stranded wire, and at least one second side element wire other than these first side element wires, and the core element wire and the first side element wires have tensile strength greater than that of the second side element wire. The core is preferably in contact with the pair of the first side element wires. The core may comprise a single core element wire or a plurality of core element wires. In the case the core comprises a plurality of core element wires, the plurality of the core element wires and the pair of first side element wires are arranged linearly in a cross-sectional view of the stranded wire.

In a second aspect, the stranded wire according to the first aspect has the core element wire and the first side element wires formed of a same material.

In a third aspect, the stranded wire according to the first aspect or second aspect is provided with a pair of the second side element wires. Preferably, the pair of the second side element wires are arranged point- symmetrically about the center of the core in a cross-sectional view of the stranded wire.

In a fourth aspect, the stranded wire according to the third aspect has the pair of the second side element wires formed of a radiopaque material.

In a fifth aspect, there is provided a guidewire including a core shaft and a coiled body covering a distal portion of the core shaft, in which the coiled body is composed of a plurality of the stranded wires according to any one of the first to fourth aspects, wound helically.

### [Advantageous Effects of Invention]

The stranded wire of the first aspect is composed of a core comprising at least one core element wire and a plurality of side element wires wound so as to cover a circumference of the core, the core and the plurality of the side element wires being stranded together. The side element wires include at least one pair of first side element wires arranged point-symmetrically about the center of the core in a cross-sectional view of the stranded wire, and at least one second side element wire other than these first side element wires. Further, the core element wire and the first side element wires have tensile strength greater than that of the second side element wire.

Accordingly, the core element wire and the first side element wires having relatively great tensile strength are arranged linearly in a cross-sectional view of the stranded wire. These core element wire and first side element wires are mutually energized due to their tensile strength, resulting in enhanced adhesion. That is, in the stranded wire, element wires linearly arranged adhere to one another strongly.

Consequently, it is possible to enhance torsional strength of the stranded wire. Thus, even in a case where a large torque is applied to the stranded wire and the stranded wire becomes greatly twisted, the stranded wire does not deform or break and safety is ensured.

Moreover, the tensile strength of one of side element wires in the stranded wire (the second side element wire) is relatively low. Generally, when a stranded wire is greatly twisted, torsional stress is caused in a circumferential direction, or compressive stress is caused by an outside element wire pressing an inside element wire, so that the stranded wire may be deformed or broken.

However, since the element wire with relatively low tensile strength (the second side element wire) is employed for some of the side element wires, the second side element wire causes the above-described torsional stress to be relieved, and the inward pressing force to be reduced. As a result, deformation or breakage of the stranded wire is suppressed, and also in this respect, safety is ensured for the stranded wire.

In the stranded wire of the second aspect, the core element wire and the first side element wires are formed of the same material. Thus, the energizing force of the core element wire and the energizing force of the first side element wires interacting with each other are equalized therebetween, so that a large torque applied to the stranded wire would seldom cause the core element wire and the first side element wires to be displaced relative to each other. Sufficient torsional strength is therefore easily ensured.

Consequently, even in a case where a large torque is applied to the stranded wire and the stranded wire becomes greatly twisted, it is possible to reliably suppress deformation or breakage of the stranded wire, thereby ensuring sufficient safety.

In the stranded wire of the third aspect, a pair of the second side element wires are provided and arranged point-symmetrically about the center of the core in a cross-sectional view of the stranded wire. Thus, torsional stress caused by a greatly twisted stranded wire is evenly relieved in a circumferential direction via the second side element wire having relatively low tensile strength. As a result, deformation or breakage of the stranded wire is effectively suppressed, and sufficient safety of the stranded wire is ensured.

In the fourth aspect, the second side element wires arranged point-symmetrically around the core element wire, respectively, is formed of the radiopaque material. Accordingly, the second side element wire formed of the radiopaque material is evenly arranged in a circumferential direction of the stranded wire, thereby making it possible to ensure sufficient visibility in a radiolucent image.

The guidewire of the fifth aspect is composed of a plurality of the stranded wires according to any one of the first to fourth aspects, wound helically. Accordingly, when pushing ahead with the guidewire along an inverted U-shaped path from the lower extremity vasculature of the right leg to the lower extremity vasculature of the left leg by, for example, the Cross-Over method, even a stranded wire that forms a coiled body and that has become greatly twisted under application of a large torque will seldom deform or break, thereby ensuring safety and making it possible to use the guidewire continuously.

### [Brief Description of Drawings]

Fig. 1 is a perspective view of a stranded wire according to a first embodiment of the present invention.
Fig. 2 is a cross-sectional view along A-A of the stranded wire shown in Fig. 1.
Fig. 3 is a schematic cross-sectional view of a stranded wire in another example according to the embodiment of the present invention.
Fig. 4 is a schematic cross-sectional view of a stranded wire in yet another example according to the embodiment of the present invention.
Fig. 5 is a cross-sectional view of a stranded wire according to a second embodiment of the present invention.
Fig. 6 is a cross-sectional view of a stranded wire according to a third embodiment of the present invention.
Fig. 7 is a cross-sectional view of a stranded wire according to a fourth embodiment of the present invention.
Fig. 8 is a cross-sectional view of a stranded wire according to a fifth embodiment of the present invention.
Fig. 9 is a cross-sectional view of a stranded wire according to a sixth embodiment of the present invention.
Fig. 10 is an enlarged cross-sectional view of a part of a guidewire according to a seventh embodiment of the present invention.
Fig. 11 is a cross-sectional view along B-B of a coiled body shown in Fig. 10.

### [Description of Embodiments]

### [First Embodiment]

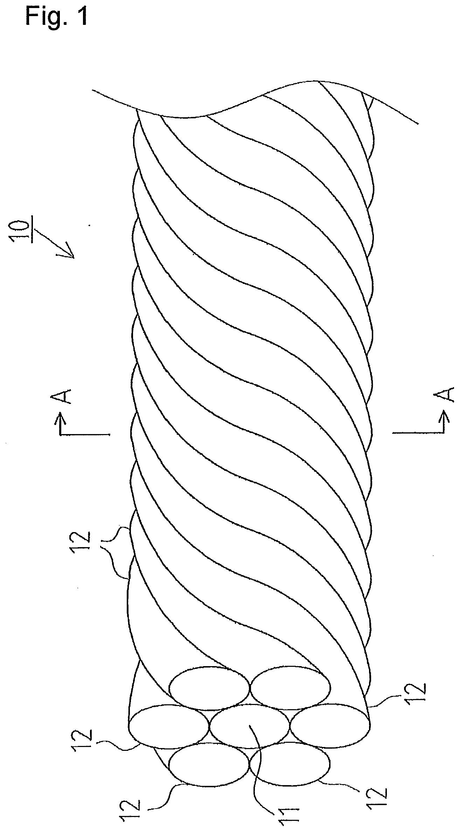

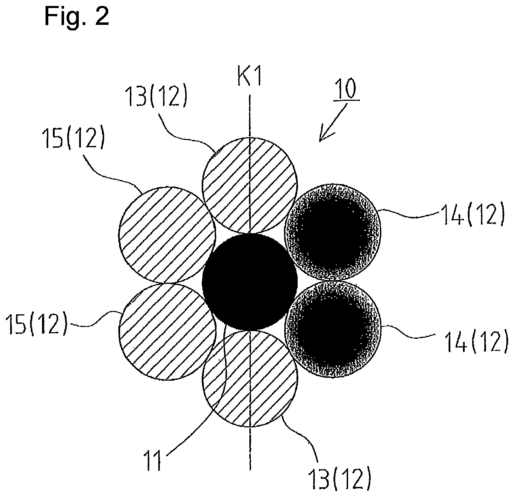

Description will be given for a stranded wire according to a first embodiment of the present invention with reference to Fig. 1 and Fig. 2. It is noted that in each figure, the stranded wire is schematically illustrated at a dimensional ratio different from an actual one.

As shown in Fig. 1 and Fig. 2, a stranded wire 10 in the present embodiment is composed of a core comprising a single core element wire 11 and six side element wires 12 wound so as to cover a circumference of the core element wire 11.

As shown in Fig. 2, the side element wires 12 include a pair of first side element wires 13 arranged point-symmetrically about the center of the core in a cross-sectional view of the stranded wire 10, and four side element wires other than the first side element wires 13. In FIG. 2, the side element wires other than the first side element wires 13 include second side element wires 14 and third side element wires 15. Virtual straight line K1 passes through the center of the core element wire 11 in a vertical direction; the second side element wires 14 are located on the right of the virtual straight line K1, and the third side element wires 15 are located on the left of the virtual straight line K1.

A material forming the core element wire 11 may be different from a material forming the first side element wires 13, and the material forming the first side element wires 13 may be the same as that forming the third side element wires 15. Moreover, the second side element wires 14 may be formed of a material different from both of the above materials forming the core element wire 11 and the first side element wires 13 (as well as the third side element wires 15).

Furthermore, the tensile strength of the core element wire 11 may be greater than the tensile strength of each of the first side element wires 13 and the third side element wires 15. Additionally, the tensile strength of the second side element wires 14 may be smaller than the tensile strength of each of the core element wire 11 and the first side element wires 13. That is, the tensile strength of the element wires forming the stranded wire 10 may increase in the order of the second side element wire 14, the first side element wire 13 (and the third side element wire 15), and the core element wire 11.

For example, the tensile strength of the core element wire 11 may be 2500 to 2700 N/mm², the tensile strength of the first side element wires 13 (and the third side element wires 15) may be 2100 to 2400 N/mm², and the tensile strength of the second side element wires 14 may be 1600 to 2000 N/mm².

The materials forming the core element wire 11 and the side element wires 12 include, for example, stainless steel, platinum, tungsten, and an Ni-Ti alloy, but are not limited thereto as long as it is possible to ensure the tensile strength increased in the order of the second side element wire 14, the first side element wire 13 (and the third side element wire 15), and the core element wire 11.

For example, SUS304WPS may be employed as the material forming the core element wire 11, SUS304WPB may be employed as the material forming the first side element wires 13 (and the third side element wires 15), and SUS304-3/4H may be employed as the material forming the second side element wires 14.

As described above, the core element wire 11 and the first side element wires 13 , each having relatively great tensile strength, are arranged linearly in a cross-sectional view. That is, the core element wire 11 and the first side element wires 13, each having relatively great tensile strength, are arranged along the virtual straight line K1 passing through the center of the core element wire 11 in a vertical direction.

The core element wire 11 and first side element wires 13 are mutually energized due to their tensile strength, resulting in enhanced mutual adhesion. Thus, in the stranded wire 10, the three element wires 11, 13, and 13 arranged linearly along the virtual straight line K1 adhere to one another strongly.

Consequently, it is possible to enhance torsional strength of the stranded wire 10. Even in a case where a large torque is applied to the stranded wire 10 and the stranded wire 10 becomes greatly twisted, the stranded wire 10 does not deform or break, and safety is ensured.

Moreover, the tensile strength of some of the side element wires (the second side element wires 14) may be lower compared to other element wires 11, 13, and 15. Generally, a stranded wire composed of a plurality of element wires stranded together has, when greatly twisted, torsional stress in a circumferential direction, and compressive stress caused by an outside element wire pressing an inside element wire (e.g., a core element wire), so that the stranded wire may be deformed or broken.

However, in the present embodiment, since an element wire with relatively low tensile strength (the second side element wire 14) is employed for some of the side element wires, the second side element wire 14 causes the above-described torsional stress to be relieved, and the inward pressing force to be reduced. As a result, deformation or breakage of the stranded wire 10 is suppressed, and also in this respect, safety is ensured for the stranded wire 10.

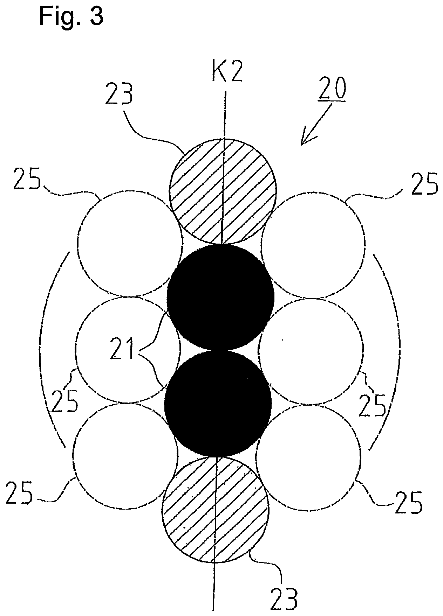

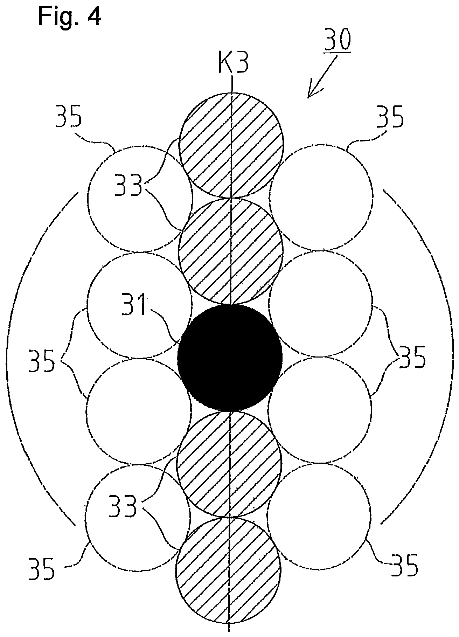

In each of the stranded wires described above, three element wires with relatively great tensile strength are arranged along a virtual straight line passing through the center of the core element wire in a vertical direction. However, the number of element wires is not limited thereto, and may be four or more (see FIGS. 3 and 4, for example).

Fig. 3 shows a stranded wire 20 having an even number (such as four or more) of element wires with relatively great tensile strength arranged along a virtual straight line K2. Apairof element wires located in the core are regarded as core element wires 21, and the other element wires are first side element wires 23. In such a conformation, at least one of side element wires 25 other than the first side element wires 23 is formed of a material with a tensile strength lower than that of the core element wires 21 and the first side element wires 23.

Fig. 4 shows a stranded wire 30 having an odd number (such as five or more) of element wires with relatively great tensile strength arranged along a virtual straight line K3 . Element wires arranged in a line, other than a core element wire 31 located in the center are first side element wires 33. That is, in this case, there are two or more pairs of the first side element wires 33 arranged along the virtual straight line K3. In such a conformation, at least one of side element wires 35 other than the first side element wires 33 is formed of a material with a tensile strength lower than that of the core element wire 31 and the first side element wires 33.

### [Second Embodiment]

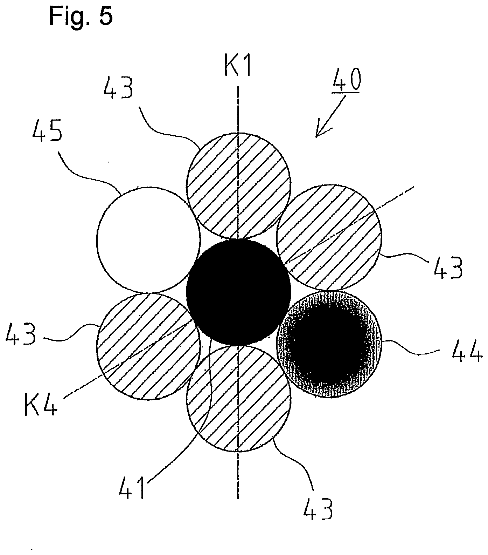

Description will be given for a stranded wire according to a second embodiment of the present invention with reference to Fig. 5. The same components as those in the above embodiment have the same reference signs so as to omit the description, and differences therebetween will be mainly described below.

In the above first embodiment, only one pair of the first side element wires are arranged point-symmetrically about the center of the core in a cross-sectional view of the stranded wire . Whereas, a stranded wire 40 of the second embodiment is provided with two pairs of first side element wires 43 arranged point-symmetrically about the center of the core in a cross-sectional view of the stranded wire 40. That is, the first side element wires 43 are arranged not only along the virtual straight line K1 but also along a virtual straight line K4 passing through the center of the core element wire 41. The element wires arranged along each of the virtual straight line K1 and the virtual straight line K4 (the core element wire 41 and the first side element wires 43) have relatively great tensile strength.

In the present embodiment, among two side element wires other than the first side element wires 43, one is a second side element wire 44, and the other is a third side element wire 45. Materials forming the core element wire 41, the first side element wires 43, the second side element wire 44, and the third side element wire 45 may be the same as those described above. That is, the tensile strength of the element wires forming the stranded wire 40 may increase in the order of the second side element wire 44, the first side element wire 43 (and the third side element wire 45), and the core element wire 41.

In a cross-sectional view of the stranded wire 40 as described above, the core element wire 41 and the first side element wires 43, each having relatively great tensile strength, are arranged along the virtual straight lines K1 and K4, respectively. The core element wire 41 and the first side element wires 43 are mutually energized due to their tensile strength, resulting in enhanced mutual adhesion. Thereby, in the stranded wire 40, the element wires 41 and 43 linearly arranged along both the virtual straight lines K1 and K4 adhere to each other strongly.

Consequently, it is possible to greatly enhance the torsional strength of the stranded wire 40, and even in a case where a large torque is applied to the stranded wire 40 and the stranded wire 40 becomes greatly twisted, safety is ensured for the stranded wire 40 without deformation or breakage.

Moreover, also in the stranded wire 40 of the present embodiment, the tensile strength of one of the side element wires (the second side element wire 44) may be lower relative to the other element wires 41, 43, and 45. Generally, a stranded wire composed of a plurality of element wires stranded together has, when greatly twisted, torsional stress in a circumferential direction, and compressive stress caused by an outside element wire pressing an inside element wire (e.g., a core element wire), so that the stranded wire may be deformed or broken.

However, since the element wire with relatively low tensile strength (the second side element wire 44) is employed for some of the side element wires of the stranded wire 40, the second side element wire 44 causes the above-described torsional stress to be relieved, and the inward pressing force to be reduced. As a result, deformation or breakage of the stranded wire 40 is suppressed, and also in this respect, safety is ensured for the stranded wire 40.

### [Third Embodiment]

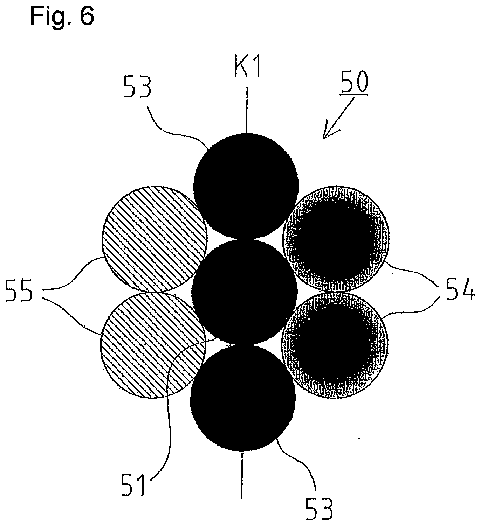

Description will be given for a stranded wire according a third embodiment of the present invention with reference to Fig. 6. The same components as those in the above embodiments have the same reference signs so as to omit the description, and differences thereamong will be mainly described below.

In the stranded wires in the above first embodiment and second embodiment, the material forming the core element wire is different from the material forming the first side element wire. Whereas, in a stranded wire 50 of the third embodiment, a core element wire 51 and first side element wires 53 are formed of the same material.

In stranded wire 50, side element wires other than the first side element wires 53 include second side element wires 54 and third side element wires 55. The virtual straight line K1 passes through the center of the core element wire 51 in a vertical direction; the second side element wires 54 are located on the right side of the virtual straight line K1, and the third side element wires 55 are located on the left side of the virtual straight line K1.

As materials, SUS3 04WPS may be employed as the material forming the core element wire 51 and the first side element wires 53, SUS304WPB may be employed as the material forming the third side element wires 55, and SUS304-3/4H may be employed as the material forming the second side element wires 54. Thus, the tensile strength of the element wires forming the stranded wire 50 may increase in the order of the second side element wire 54, the third side element wire 55, and the core element wire 51 (and the first side element wire 53) .

Sufficient torsional strength is therefore ensured for the stranded wire 50, even in a case where a large torque is applied to the stranded wire 50 and the stranded wire 50 becomes greatly twisted. Thus, the stranded wire 50 is seldom deformed or broken, and safety is ensured.

Additionally, in the present embodiment where the core element wire 51 and the first side element wires 53 are formed of the same material, the energizing force of the core element wire 51 and the energizing force of the first side element wires 53 interacting with each other are equalized therebetween, so that a large torque applied to the stranded wire 50 does not cause the core element wire 51 and the first side element wire 53 to be displaced relative to each other. Sufficient torsional strength is thereby easily ensured.

Consequently, even in a case where a large torque is applied to the stranded wire 50 and the stranded wire 50 becomes greatly twisted, it is possible to reliably suppress deformation or breakage of the stranded wire 50, thereby ensuring sufficient safety.

### [Fourth Embodiment]

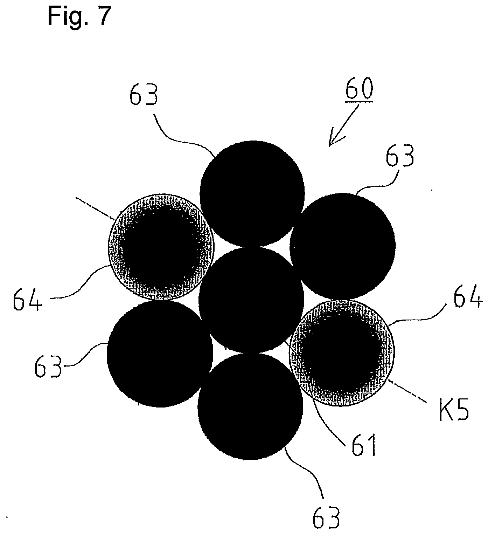

Description will be given for a stranded wire according to a fourth embodiment of the present invention with reference to Fig. 7. The same components as those in the above embodiments have the same reference signs so as to omit the description, and differences thereamong will be mainly described below.

In the above third embodiment, the second side element wires (the wire with relatively small tensile strength) are arranged only on one side across the virtual straight line passing through the center of the core element wire to extend in a vertical direction. That is, the second side element wires are unevenly located in a circumferential direction of the stranded wire. Whereas, in a stranded wire 60 of the fourth embodiment, the pair of second side element wires 64 are arranged point-symmetrically about the center of the core in a cross-sectional view of the stranded wire 60.

In the present embodiment, the second side element wires 64 are the only pair of side element wires positioned along a virtual straight line K5 passing through the center of the core element wire 61, and all the side element wires other than these second side element wires 64 are first side element wires 63. Materials forming the core element wire 61, the first side element wires 63, and the second side element wires 64 may be the same as described above. That is, the tensile strength of the second side element wires 64 may be smaller compared to that of the core element wire 61 and the first side element wires 63.

Accordingly, sufficient torsional strength is ensured for the stranded wire 60 even where a large torque is applied to the stranded wire 60 and the stranded wire has become greatly twisted. Safety is therefore ensured for the stranded wire 60 without deformation or breakage.

Furthermore, where the second side element wires 64 with relatively small tensile strength are point-symmetrically arranged, the torsional stress caused when the stranded wire 60 is greatly twisted is relieved evenly in a circumferential direction via the second side element wire 64. As a result, deformation or breakage of the stranded wire 60 is effectively suppressed, so that sufficient safety is ensured for the stranded wire 60.

### [Fifth Embodiment]

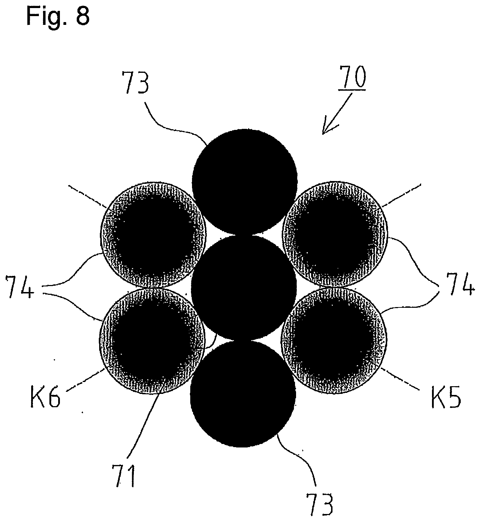

Description will be given for a stranded wire according to a fifth embodiment of the present invention with reference to Fig. 8. The same components as those in the above embodiments have the same reference signs so as to omit the description, and differences thereamong will be mainly described below.

In the above fourth embodiment, the second side element wires 64 are the only pair of side element wires along the virtual straight line K5 passing through the center of the core element wire. Whereas, in a stranded wire 70 of the fifth embodiment, second side element wires 74 are positioned not only along the virtual straight line K5, but also along a virtual straight line K6 passing through the center of a core element wire 71. That is, in the stranded wire 70, all the side element wires excluding first side element wires 73 are the second side element wires 74. Materials forming the core element wire 71, the first side element wires 73, and the second side element wires 74 may be the same as those described above. That is, the tensile strength of the second side element wires 74 may be smaller compared to that of the core element wire 71 and the first side element wires 73.

Accordingly, the torsional strength of the stranded wire 70 is enhanced as with the above embodiments, and a plurality of the second side element wires 74 cause the torsional stress in a circumferential direction to be sufficiently relieved. As a result, it is possible to suppress deformation or breakage of the stranded wire 70 more effectively, thereby ensuring sufficient safety for the stranded wire 70.

### [Sixth Embodiment]

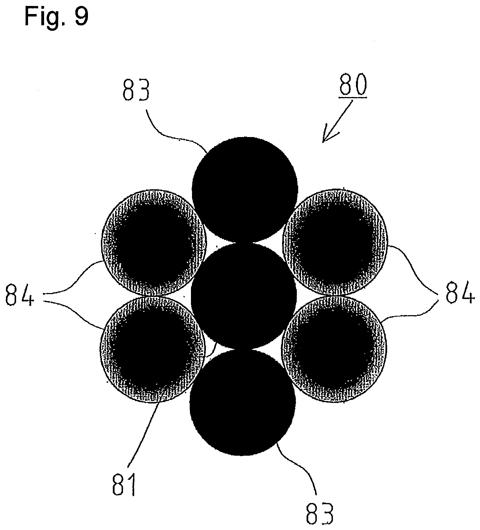

Description will be given for a stranded wire according to a sixth embodiment of the present invention with reference to Fig. 9. The same components as those in the above embodiments have the same reference signs so as to omit the description, and differences thereamong will be mainly described below.

In the above fifth embodiment, the second side element wires may be formed of a radiolucent material, such as stainless steel. Whereas, in a stranded wire 80 of FIG. 9, second side element wires 84 are formed of a radiopaque material. That is, in the stranded wire 80, two pairs of the second side element wires 84, which are respectively arranged point-symmetrically about the center of the core in a cross-sectional view of the stranded wire 80, are formed of a radiopaque material.

Materials forming the second side element wires 84 include, for example, gold, platinum, tungsten, an alloy containing these elements (for example, a platinum-nickel alloy), or the like. It is preferable to employ a platinum-nickel alloy in terms of ensuring a tensile strength smaller than that of both the core element wire 81 and a first side element wires 83.

In this way, in the stranded wire 80 of the present embodiment, the second side element wires 84, which are arranged point-symmetrically about the center of the core, are formed of the radiopaque material. Accordingly, since the second side element wires 84 formed of the radiopaque material are evenly arranged in a circumferential direction of the stranded wire 80, it is possible to sufficiently ensure visibility in a radiolucent image even while the stranded wire 80 is being rotated.

### [Seventh Embodiment]

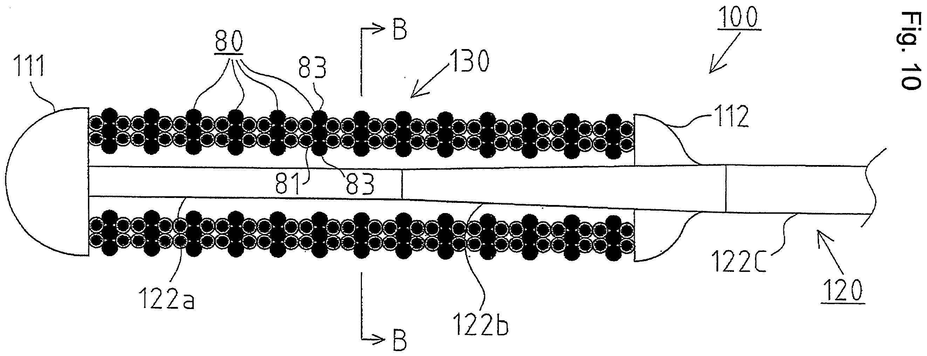

Fig. 10 is an enlarged cross-sectional view showing a part of a guidewire employing the stranded wire of the present invention. In Fig. 10, a distal side of the guidewire to be inserted into a body is provided on the left, and a proximal side of the guidewire to be operated by a manipulator such as a doctor is provided on the right. Fig. 10 shows a guidewire employing a coiled body composed of the stranded wire of the sixth embodiment. It is noted that, in this figure, the guidewire including a cross-sectional shape of the coiled body formed of the stranded wire is schematically illustrated at a dimensional ratio different from the actual one.

The guidewire 100 shown in Fig. 10 is used for treatment of the lower extremity vasculature by, for example, the Cross-Over method. The guidewire 100 includes a core shaft 120, and a coiled body 130 covering a circumference of a distal portion of the core shaft 120.

First, description will be given for the core shaft 120. The core shaft 120 includes a small diameter portion 122a, a tapered portion 122b, and a large diameter portion 122c in order from the distal end to the proximal end. The small diameter portion 122a is positioned at the distal end of the core shaft 120 and is the most flexible part of the core shaft 120. The small diameter portion 122a is formed in a tabular shape by press working. The tapered portion 122b has a cross section formed in a tapered round shape whose diameter is gradually reduced toward the distal end. The large diameter portion 122c has a diameter larger than that of the small diameter portion 122a.

The core shaft 120 is formed usingmaterials such as, for example, stainless steel (SUS304), a super elastic alloy such as an Ni-Ti alloy, a piano wire and a cobalt-based alloy. However, the material are not especially limited thereto.

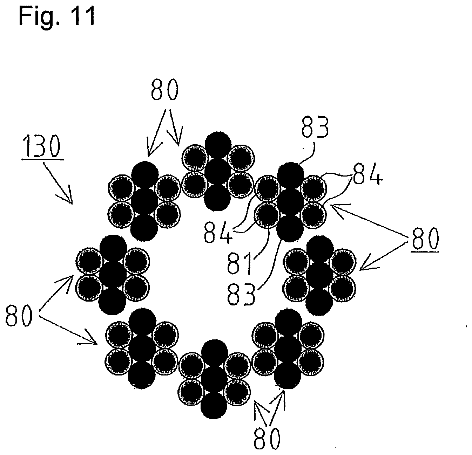

Next, description will be given for the coiled body 130. As shown in Fig. 11, the coiled body 130 is composed of a plurality of the stranded wire 80 of the sixth embodiment (eight stranded wires 80 in the present embodiment) wound helically.

A distal end of the coiled body 130 is fixed to the distal end of the core shaft 120 with a distal joint portion 111. On the other hand, a proximal end of the coiled body 130 is fixed to the core shaft 120 with a proximal joint portion 112 . Materials forming the distal joint portion 111 and the proximal joint portion 112 include, for example, a brazing metal such as an Sn-Pb alloy, a Pb-Ag alloy, an Sn-Ag alloy and an Au-Sn alloy.

When pushing ahead with the guidewire 100 along an inverted U-shaped path from the lower extremity vasculature of the right leg to the lower extremity vasculature of the left leg by, for example, the Cross-Over method, a large torque is applied to the coiled body 130, and the coiled body 130 may therefore become greatly twisted.

At the time, with the guidewire 100 of the present embodiment, the stranded wire 80 forming the coiled body 130 has the element wires 81, 83, and 83 with relatively great tensile strength arranged linearly in a cross-sectional view, and the torsional strength of the stranded wire 80 is thus enhanced. As a result, even in a case where a large torque is applied to the coiled body 130 and the coil body 130 becomes greatly twisted, safety is ensured without deformation or breakage of the coil body 130. Therefore, it is possible to use the guidewire 100 continuously.

Further, the tensile strength of some of the side element wires (the second side element wires 84) is relatively low. Generally, such a stranded wire has, when greatly twisted, a torsional stress in a circumferential direction, or compressive stress caused by an outside element wire pressing an inside element wire, so that the stranded wire may be deformed or broken.

However, in the present embodiment, since element wires with relatively low tensile strength (the second side element wires 84) are employed for some of the side element wires, the above-described torsional stress is relieved, and the inward pressing force is reduced. As a result, deformation or breakage of the coiled body 130 is suppressed, and also in this respect, safety is ensured for the coiled body 130.

Additionally, two pairs of the second side element wires 84, which are respectively arranged point-symmetrically about the center of the core in a cross-sectional view of the stranded wire 80, are formed of the radiopaque material. Thereby, since the second side element wires 84 formed of the radiopaque material are evenly arranged in a circumferential direction of the stranded wire 80, it is possible to ensure sufficient visibility in a radiolucent image even while the guidewire 100 is inserted deep into a blood vessel and is being rotated.

The guidewire employing the stranded wire of the sixth embodiment has been described in the present embodiment, however, also for the guidewire employing the stranded wire in the first embodiment to fifth embodiment, it is possible to suppress deformation or breakage of a coiled body as with the present embodiment, thereby making it possible to ensure safety and allowing continuous use.

### [References Signs List]

10, 20, 30, 40, 50, 60, 70, 80...stranded wire; 11, 21, 31, 41, 51, 61, 71, 81...core element wire; 12...side element wire; 13, 23, 33, 43, 53, 63, 73, 83...first side element wire; 14, 44, 54, 64, 74, 84...second side element wire; 100...guidewire; 120...core shaft; and 130...coiled body.

## Claims

1. A stranded wire (10, 20, 30, 40, 50, 60, 70, 80) comprising a core comprising a single core element wire (11, 21, 31, 41, 51, 61, 71, 81) and a plurality of side element wires (12) wound so as to cover a circumference of the core, the core and the plurality of the side element wires being stranded together, wherein
the side element wires (12) include at least one pair of first side element wires (13, 23, 33, 43, 53, 63, 73, 83), and at least one second side element wire (14, 44, 54, 64, 74, 84) other than these first side element wires, **characterized in that**
the pair of first side element wires (13, 23, 33, 43, 53, 63, 73, 83) is arranged point-symmetrically about the center of the core in a cross-sectional view of the stranded wire, and
the core element wire (11, 21, 31, 41, 51, 61, 71, 81) and the first side element wires (13, 23, 33, 43, 53, 63, 73, 83) have tensile strength greater than that of the second side element wire (14, 44, 54, 64, 74, 84).

2. The stranded wire (10, 20, 30, 40, 50, 60, 70, 80) according to claim 1, wherein
the core is in contact with the pair of the first side element wires (13, 23, 33, 43, 53, 63, 73, 83).

3. The stranded wire (50, 60, 70, 80) according to claim 1 or 2, wherein
the core element wire (51, 61, 71, 81) and the first side element wires (53, 63, 73, 83) are formed of a same material.

4. The stranded wire (10, 20, 30, 40) according to claim 1 or 2, wherein
tensile strength of the core element wire (11, 21, 31, 41), the pair of first side element wires (13, 23, 33, 43), and the second side element wire (14, 25, 35, 44) increases in the order of the second side element wire (14, 25, 35, 44), the first side element wire (13, 23, 33, 43), and the core element wire (11, 21, 31, 41) .

5. The stranded wire (20, 30, 50, 60, 70, 80) according to any one of claims 1 to 4 comprising
at least one pair of second side element wires (25, 35, 54, 64, 74, 84).

6. The stranded wire (60, 70, 80) according to claim 5, wherein
the pair of the second side element wires (64, 74, 84) are arranged point-symmetrically about the center of the core in a cross-sectional view of the stranded wire (60, 70, 80).

7. The stranded wire (80) according to claim 6, wherein
the pair of the second side element wires (84) are formed of a radiopaque material.

8. The stranded wire (80) according to claim 6 or 7, wherein the core element wire (81) and the first side element wire (83) are formed of stainless steel and the second side element wires (84) are formed of platinum-nickel alloy.

9. A guidewire (100), comprising:
a core shaft (120); and
a coiled body (130) covering a distal portion of the core shaft (120), wherein
the coiled body (130) is composed of a plurality of the stranded wires (10, 20, 30, 40, 50, 60, 70, 80) according to any one of claims 1 to 8, wound helically.

## Patentansprüche

1. Litzendraht (10, 20, 30, 40, 50, 60, 70, 80) mit einem Kern, der einen einzelnen Kernelementdraht (11, 21, 31, 41, 51, 61, 71, 81) umfasst, und einer Vielzahl von Seitenelementdrähten (12), die um den Kern herum gewickelt sind, wobei der Kern und die Vielzahl von Seitenelementdrähten miteinander verdrillt sind, wobei
die Seitenelementdrähte (12) wenigstens ein Paar erste Seitenelementdrähte (13, 23, 33, 43, 53, 63, 73, 83) und wenigstens einen von den ersten Seitenelementdrähten verschiedenen zweiten Seitenelementdraht (14, 44, 54, 64, 74, 84) umfassen, **dadurch gekennzeichnet, dass**
die beiden ersten Seitenelementdrähte (13, 23, 33, 43, 53, 63, 73, 83) in einer Querschnittansicht des Litzendrahts bezüglich der Kernmitte punktsymmetrisch angeordnet sind, und
der Kernelementdraht (11, 21, 31, 41, 51, 61, 71, 81) und die ersten Seitenelementdrähte (13, 23, 33, 43, 53, 63, 73, 83) eine höhere Zugfestigkeit haben als der zweite Seitenelementdraht (14, 44, 54, 64, 74, 84).

2. Litzendraht (10, 20, 30, 40, 50, 60, 70, 80) nach Anspruch 1, wobei der Kern mit den beiden ersten Seitenelementdrähten (13, 23, 33, 43, 53, 63, 73, 83) in Kontakt ist.

3. Litzendraht (50, 60, 70, 80) nach Anspruch 1 oder 2, wobei der Kernelementdraht (51, 61, 71, 81) und die ersten Seitenelementdrähte (53, 63, 73, 83) aus dem gleichen Material gebildet sind.

4. Litzendraht (10, 20, 30, 40) nach Anspruch 1 oder 2, wobei die Zugfestigkeit des Kernelementdrahts (11, 21, 31, 41), der beiden ersten Seitenelementdrähte (13, 23, 33, 43) und des zweiten Seitenelementdrahts (14, 25, 35, 44) in der Reihenfolge zweiter Seitenelementdraht (14, 25, 35, 44), erster Seitenelementdraht (13, 23, 33, 43) und Kernelementdraht (11, 21, 31, 41) zunimmt.

5. Litzendraht (20, 30, 50, 60, 70, 80) nach einem der Ansprüche 1 bis 4, mit wenigstens einem Paar zweiter Seitenelementdrähte (25, 35, 54, 64, 74, 84).

6. Litzendraht (60, 70, 80) nach Anspruch 5, wobei die beiden zweiten Seitenelementdrähte (64, 74, 84) in einer Querschnittansicht des Litzendrahts (60, 70, 80) bezüglich der Kernmitte punktsymmetrisch angeordnet sind.

7. Litzendraht (80) nach Anspruch 6, wobei die beiden zweiten Seitenelementdrähte (84) aus einem röntgenopaken Material gebildet sind.

8. Litzendraht (80) nach Anspruch 6 oder 7, wobei der Kernelementdraht (81) und der erste Seitenelementdraht (83) aus Edelstahl und die zweiten Seitenelementdrähte (84) aus einer Platin-Nickel-Legierung gebildet sind.

9. Führungsdraht (100) mit:
einer Kernschaft (120); und
einem Wicklungskörper (130), der einen distalen Abschnitt des Kernschafts (120) umhüllt, wobei
der Wicklungskörper (130) aus einer Vielzahl von schraubenförmig gewickelten Litzendrähten (10, 20, 30, 40, 50, 60, 70, 80) nach einem der Ansprüche 1 bis 8 zusammengesetzt ist.

## Revendications

1. Fil toronné (10, 20, 30, 40, 50, 60, 70, 80) comprenant un noyau comprenant un fil d'élément de noyau unique (11, 21, 31, 41, 51, 61, 71, 81) et une pluralité de fils d'élément latéraux (12) enroulés afin de recouvrir une circonférence du noyau, le noyau et la pluralité de fils d'élément latéraux étant toronnés ensemble, dans lequel :
les fils d'élément latéraux (12) comprennent au moins une paire de premiers fils d'élément latéraux (13, 23, 33, 43, 53, 63, 73, 83) et au moins un second fil d'élément latéral (14, 44, 54, 64, 74, 84) différent de ces premiers fils d'élément latéraux, **caractérisé en ce que** :
la paire de premiers fils d'élément latéraux (13, 23, 33, 43, 53, 63, 73, 83) est agencée de manière symétrique en pointe autour du centre du noyau sur une vue transversale du fil toronné, et
le fil d'élément de noyau (11, 21, 31, 41, 51, 61, 71, 81) et les premiers fils d'élément latéraux (13, 23, 33, 43, 53, 63, 73, 83) ont une résistance à la tension supérieure à celle du second fil d'élément latéral (14, 44, 54, 64, 74, 84).

2. Fil toronné (10, 20, 30, 40, 50, 60, 70, 80) selon la revendication 1, dans lequel :
le noyau est en contact avec la paire de premiers fils d'élément latéraux (13, 23, 33, 43, 53, 63, 73, 83).

3. Fil toronné (50, 60, 70, 80) selon la revendication 1 ou 2, dans lequel :
le fil d'élément de noyau (51, 61, 71, 81) et les premiers fils d'élément latéraux (53, 63, 73, 83) sont formés avec un même matériau.

4. Fil toronné (10, 20, 30, 40) selon la revendication 1 ou 2, dans lequel :
la résistance à la tension du fil d'élément de noyau (11, 21, 31, 41), de la paire de premiers fils d'élément latéraux (13, 23, 33, 43) et du second fil d'élément latéral (14, 25, 35, 44) augmente dans l'ordre du second fil d'élément latéral (14, 25, 35, 44), du premier fil d'élément latéral (13, 23, 33, 43) et du fil d'élément de noyau (11, 21, 31, 41).

5. Fil toronné (20, 30, 50, 60, 70, 80) selon l'une quelconque des revendications 1 à 4, comprenant :
au moins une paire de seconds fils d'élément latéraux (25, 35, 54, 64, 74, 84).

6. Fil toronné (60, 70, 80) selon la revendication 5, dans lequel :
la paire de seconds fils d'élément latéraux (64, 74, 84) sont agencés de manière symétrique en pointe autour du centre du noyau sur une vue transversale du fil toronné (60, 70, 80).

7. Fil toronné (80) selon la revendication 6, dans lequel :
la paire de seconds fils d'élément latéraux (84) est formée avec un matériau radio-opaque.

8. Fil toronné (80) selon la revendication 6 ou 7, dans lequel le fil d'élément de noyau (81) et le premier fil d'élément latéral (83) sont formés avec de l'acier inoxydable et les seconds fils d'élément latéraux (84) sont formés avec un alliage de platine - nickel.

9. Fil-guide (100) comprenant :
un arbre de noyau (120) ; et
un corps hélicoïdal (130) recouvrant une partie distale de l'arbre de noyau (120), dans lequel :
le corps hélicoïdal (130) est composé d'une pluralité de fils toronnés (10, 20, 30, 40, 50, 60, 70, 80) selon l'une quelconque des revendications 1 à 8, enroulés de manière hélicoïdale.
